(19) **Europäisches Patentamt European Patent Office Office européen des brevets**

(11) **EP 3 896 135 B1**

(12) **EUROPEAN PATENT SPECIFICATION**

(45) Date of publication and mention
of the grant of the patent:
**03.12.2025 Bulletin 2025/49**

(21) Application number: **19896317.5**

(22) Date of filing: **10.12.2019**

(51) International Patent Classification (IPC):
*C09J 7/38* (2018.01)    *C09J 133/08* (2006.01)
*A61L 15/34* (2006.01)    *A61L 15/58* (2006.01)
*A61F 13/0246* (2024.01)

(52) Cooperative Patent Classification (CPC):
(C-Sets available)
**C09J 7/385; A61F 13/0253; A61L 15/34;
A61L 15/585; C09J 133/08;** C08F 220/1809
(Cont.)

(86) International application number:
**PCT/JP2019/048250**

(87) International publication number:
**WO 2020/122058 (18.06.2020 Gazette 2020/25)**

(54) **TAPE OR SHEET FOR SKIN ATTACHMENT**

BAND ODER FOLIE ZUR HAUTBEFESTIGUNG

BANDE OU FEUILLE POUR FIXATION À LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.12.2018 JP 2018232674**

(43) Date of publication of application:
**20.10.2021 Bulletin 2021/42**

(73) Proprietor: **NITTO DENKO CORPORATION
Ibaraki-shi
Osaka 567-8680 (JP)**

(72) Inventors:
• **YOSHIDA, Noboru
Ibaraki-shi, Osaka 567-8680 (JP)**
• **ABE, Eriko
Ibaraki-shi, Osaka 567-8680 (JP)**
• **ARIMA, Ryuhei
Ibaraki-shi, Osaka 567-8680 (JP)**

(74) Representative: **Grünecker Patent- und
Rechtsanwälte
PartG mbB
Leopoldstraße 4
80802 München (DE)**

(56) References cited:
**EP-A2- 1 184 039          WO-A1-01/43729
WO-A1-2014/021052      JP-A- 2002 065 841
JP-A- 2015 520 247        US-A1- 2009 274 748**

• **POLYMERDATABASE.COM: "Rosins",
POLYMER PROPERTIES DATABASE, 8 July 2018
(2018-07-08), pages 1 - 3, XP055946183,
Retrieved from the Internet <URL:https://web.
archive.org/web/20180708031042/https://
polymerdatabase.com/polymer%20classes/
Rosin.html> [retrieved on 20220726]**
• **HARDHIANTI MEIGA PUTRI WAHYU ET AL:
"Kinetic Studies of Esterification of Rosin and
Pentaerythritol", PROCESSES, vol. 10, no. 1, 25
January 2022 (2022-01-25), CH, pages 39,
XP055946256, ISSN: 2227-9717, DOI: 10.3390/
pr10010039**

(52) Cooperative Patent Classification (CPC): (Cont.)

C-Sets
**A61L 15/585, C08L 33/10;**
**A61L 15/585, C08L 93/04;**
C08F 220/1809, C08F 220/281, C08F 220/06

## Description

Technical Field

The present invention relates to a tape or sheet for sticking to skin.

Background Art

A tape or sheet for sticking to skin is used for the purpose of protecting a skin surface by sticking to the skin surface or fixing a catheter, medical instruments or the like to the skin surface (see, for example, Patent Document 1). Further examples of adhesive tapes or sheets for application to skin are disclosed in Patent Documents 2 and 3.

Citation List

Patent Documents

Patent Document 1: JP-A-2007-209515
Patent Document 2: EP 1 184 039 A2
Patent Document 3: US 2009/274748 A1

Summary of Invention

Technical Problem

Skin surface generally has irregular and complicated structure. Therefore, it has been considered to improve adhesion of a tape or sheet for sticking to skin to the skin surface by improving flexibility of an adhesive layer constituting the tape or sheet for sticking to skin. However, when flexibility of the adhesive layer is excessively improved, cohesive force of the adhesive layer is decreased. As a result, the adhesive sometimes remains on a skin surface when peeling and removing the tape or sheet for sticking to skin from the skin surface, and this may cause adhesive residue.

Furthermore, when a tape or sheet for sticking to skin is maintained over a long period of time in the state of sticking to a skin surface, the tape or sheet for sticking to skin sometimes easily peels from the skin by, for example, sweat generated from the skin. On the other hand, when adhesive force of the tape or sheet for sticking to skin is set to be excessively high in order to prevent peeling of the tape or sheet, skin irritation, dermatitis or the like may be caused to delicate skin surface when peeling the tape or sheet from the skin surface.

Thus, the tape or sheet for sticking to skin is required to maintain exquisite balance in internal cohesive force of the adhesive layer, adhesive force to skin, and the like. However, it was difficult to say that the conventional tape or sheet for sticking to skin has sufficient level of characteristics.

In view of the above current state, the present invention has an object to provide a tape or sheet for sticking to skin, that has good stickiness to a skin surface, particularly stickiness to a skin surface when bathing or on which water such as sweat is present, can prevent adhesive residue or anchor destruction when peeling and removing from a skin surface, can reduce skin irritation when peeling and removing from a skin surface, and additionally has excellent moisture permeability.

Solution to Problem

As a result of intensive investigations to achieve the above objects, the present inventors have found that in a tape or sheet for sticking to skin including a base material and an adhesive layer laminated on at least one surface of the base material, when the adhesive layer contains an acrylic copolymer obtained from a specific monomer composition, a component that is liquid or paste-like at room temperature, and a tackifier, and further the tackifier contains a rosin ester, flexibility and cohesive property can be imparted to the adhesive layer, and additionally high moisture permeability can be obtained, and completed the present invention.

The present invention based on the above findings is described below.

(1) A tape or sheet for sticking to skin, which comprises a base material and an adhesive layer laminated on at least one surface of the base layer, wherein the adhesive layer contains an acrylic copolymer obtained by copolymerizing a monomer mixture including a (meth)acrylic acid alkyl ester monomer and an alkoxy group-containing ethylenically unsaturated monomer, a component that is liquid or paste-like at room temperature, and a tackifier, wherein the tackifier includes a rosin ester, wherein a content of the rosin ester in the adhesive layer is 3 parts by mass or more and 50 parts by mass or less based on 100 parts by mass of the acrylic copolymer,

and wherein the component that is liquid or paste-like at room temperature includes a carboxylic acid ester.

(2) The tape or sheet for sticking to skin described in (1), wherein the carboxylic acid ester includes a glycerin ester of a saturated fatty acid.

(3) The tape or sheet for sticking to skin described in (1) or (2), wherein a softening point of the rosin ester is 50 to 160°C, the softening point being measured according to JIS K 5601-2-2 (ring and ball method).

Effects of the Invention

The tape or sheet for sticking to skin of the present invention has excellent stickiness to a skin surface, particularly excellent stickiness to a skin surface when bathing or on which water such as sweat is present, can prevent adhesive residue or anchor destruction when peeling and removing from a skin surface, and can reduce skin irritation when peeling and removing from a skin surface. Additionally, the tape or sheet has excellent moisture permeability and can suppress stuffiness, itch and the like when sticking, and therefore is useful in sticking over a long period of time.

Brief Description of Drawings

[FIG 1]

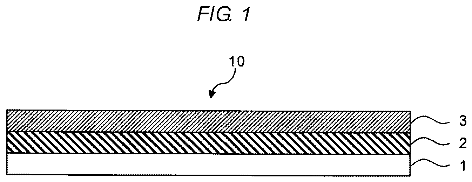

FIG 1 is schematic cross-sectional view showing one aspect of a tape or sheet for sticking to skin, having a release liner.
[FIG 2]

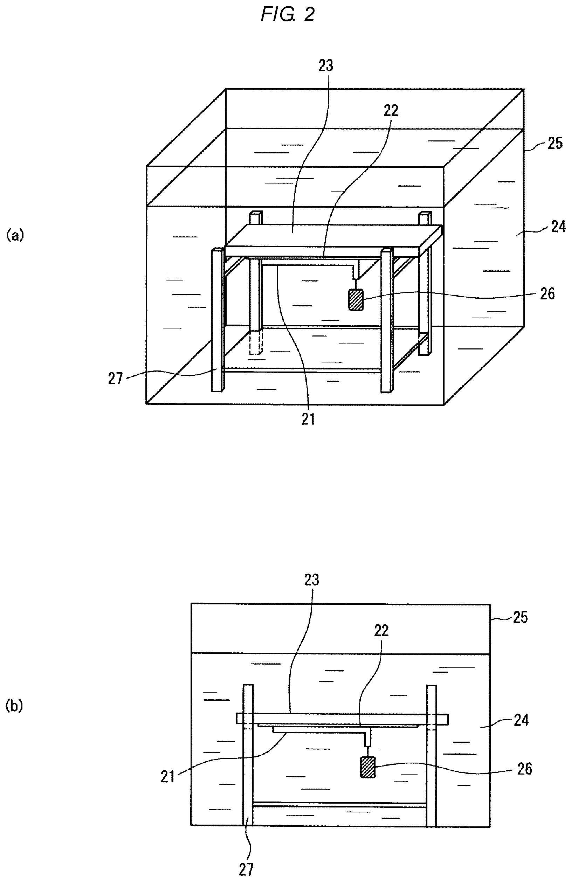

FIG 2 is a view showing a measurement method of underwater constant load peelability of a tape for sticking to skin, wherein (a) is its schematic perspective view and (b) is its schematic front view. Each dimension in the Figure is sometimes described exaggeratedly in order to clarify the characteristics of the present invention.

Description of Embodiments

Preferred embodiments of the present invention are described below, but the present invention is not construed as being limited to those specific embodiments.

In the following drawings, it is sometimes explained by allotting the same reference signs to members and parts exhibiting the same actions, and overlapping explanations are sometimes omitted or simplified. The embodiments described in the drawings do not always precisely indicate size and scale of the actual product.

The present invention is a tape or sheet for sticking to skin, including a base material and an adhesive layer laminated on at least one surface of the base material, wherein the adhesive layer contains an acrylic copolymer obtained by copolymerizing a monomer mixture including a (meth)acrylic acid alkyl ester monomer and an alkoxy group-containing ethylenically unsaturated monomer, a component that is liquid or paste-like at room temperature, and a tackifier, wherein the component that is liquid or paste-like at room temperature includes a carboxylic acid ester, wherein the tackifier includes a rosin ester, and wherein a content of the rosin ester in the adhesive layer is 3 parts by mass or more and 50 parts by mass or less based on 100 parts by mass of the acrylic copolymer.

The (meth)acrylic acid alkyl ester monomer constituting the acrylic copolymer is a component that imparts stickiness to skin to the adhesive layer, and use of a long chain alkyl ester having an alkyl group containing 4 or more and more preferably 6 to 18 carbon atoms is effective. The adhesive containing the acrylic copolymer has the advantages that contamination of impurities is less as compared with a rubber-base adhesive, irritation to skin is less and deterioration of adhesive force is difficult to occur even by the use for a long period of time.

As the (meth)acrylic acid alkyl ester, for example, the alkyl group includes butyl, pentyl, hexyl, heptyl, octyl, 2-ethylhexyl, nonyl, isononyl, decyl, undecyl, dodecyl and tridecyl. Above all, 2-ethylhexyl and isononyl are preferably used. Those can be used alone or by combining two or more kinds, and an alkyl ester chain of those may be any of a straight chain or a branched chain.

The alkoxy group-containing ethylenically unsaturated monomer is a component that imparts water vapor permeability, that is, moisture permeability, to the acrylic copolymer. By imparting moisture permeability to the acrylic copolymer, consequently the adhesive layer containing the acrylic copolymer, stuffiness, itch and the like during sticking the tape or sheet for sticking to skin can be suppressed, and such a tape or sheet is useful to the sticking for a long period of time. The alkoxy group-containing ethylenically unsaturated monomer is not particularly limited, but includes alkoxyalkyl acrylates having an alkoxy group having 1 to 4 carbon atoms, such as methoxypolyethylene glycol acrylate, ethoxydiethylene glycol acrylate, butoxydiethylene glycol acrylate, methoxyethyl acrylate, ethoxyethyl acrylate and butoxyethyl acrylate. Above all, methoxyethyl acrylate is preferably used.

The acrylic copolymer in the present invention preferably uses an acrylic copolymer obtained from a monomer mixture further containing a carboxyl group-containing ethylenically unsaturated monomer. By containing the carboxyl group-containing ethylenically unsaturated monomer, the improvement in cohesive force of the copolymer obtained can be expected. The carboxyl group-containing ethylenically unsaturated monomer is not particularly limited, but, for example, includes (meth)acrylic acid, itaconic acid, crotonic acid, fumaric acid and (anhydrous) maleic acid. Above all, (meth)acrylic acid is preferably used in copolymerizability and handleability.

In the acrylic copolymer in the present invention, other than each monomer described above, a monomer such as styrene, vinyl acetate or N-vinyl-2-pyrrolidone may be appropriately copolymerized as necessary.

The content of the (meth)acrylic acid alkyl ester monomer in the acrylic copolymer is that the upper limit is preferably 80 mass % and more preferably 75 mass % and the lower limit is preferably 40 mass % and more preferably 50 mass %. When the content of the (meth)acrylic acid alkyl ester monomer is in the above range, good skin stickiness is exhibited, and the effect that adhesive residue phenomenon to the skin does not cause when peeling from the skin and excellent peelability is exhibited is more satisfactorily obtained.

The content of the alkoxy group-containing ethylenically unsaturated monomer in the acrylic acid is that the upper limit is preferably 60 mass % and more preferably 50 mass % and the lower limit is preferably 10 mass %, more preferably 20 mass % and still more preferably 25 mass %. Incidentally, when the carboxyl group-containing ethylenically unsaturated monomer is contained in the acrylic copolymer, the content of the alkoxy group-containing ethylenically unsaturated monomer is that the upper limit is preferably 50 mass % and more preferably 45 mass % and the lower limit is preferably 10 mass % and more preferably 20 mass %. When the content of the alkoxy group-containing ethylenically unsaturated monomer is in the above range, excellent moisture permeability can be imparted to the adhesive layer, and the effect that stuffiness, itch sensation and the like can be suppressed during sticking to a skin surface is more satisfactorily obtained.

When the carboxyl group-containing ethylenically unsaturated monomer is contained in the acrylic copolymer, the content of the carboxyl group-containing ethylenically unsaturated monomer is that the upper limit is preferably 10 mass % and more preferably 8 mass % and the lower limit is preferably 1 mass % and more preferably 3 mass %. When the carboxyl group-containing ethylenically unsaturated monomer is in the above range, excellent cohesive force can be imparted to the adhesive layer, adhesive residue phenomenon and the like can be suppressed, and additionally the effect that skin irritation can be suppressed is more satisfactorily obtained.

The acrylic copolymer in the present invention can be obtained by the conventional polymerization methods such or a solution polymerization method, an emulsion polymerization method or a suspension polymerization. The acrylic copolymer can be obtained by conducting radical polymerization using a radical polymerization initiator such as a peroxide compound or an azo compound.

The acrylic copolymer is preferably that glass transition temperature thereof is 250°K or lower. In other words, when the acrylic copolymer has the glass transition temperature of 250°K or lower, the tape or sheet for sticking to skin is easy to have sufficient skin adhesiveness when sticking the tape or sheet to a skin surface.

The weight average molecular weight of a soluble component to a molecular weight measuring solvent of the acrylic copolymer is desirably adjusted to preferably 500,000 to 2,500,000, more preferably 600,000 to 2,000,000 and still more preferably 600,000 to 1,000,000. The weight average molecular weight and molecular weight distribution used herein are values measured using gel permeation chromatography (GPC). Measuring sample is dissolved in tetrahydrofuran, and a soluble element passing through a membrane filter having a diameter of 0.45 $\mu$m is measured and calculated in terms of polystyrene.

The adjustment of the glass transition temperature and weight average molecular weight of the acrylic copolymer can be conducted by appropriately controlling polymerization conditions (monomer species, blending ratio of monomer, solid content ratio, reaction temperature, reaction time and the like) of the acrylic copolymer.

The adhesive layer in the present invention contains a component that is liquid or paste-like at room temperature (25°C) (hereinafter simply referred to as "liquid or past-like component"). This component imparts the characteristics that flexibility is imparted to the adhesive layer, good adhesiveness to a skin surface is maintained, damage of skin stratum corneum is less when peeling and skin irritation is reduced.

The component that is liquid or paste-like at room temperature includes a carboxylic acid ester. For example, esters between various acids and polyhydric alcohol such as ethylene glycol or glycerin, and the like can be used. More specifically, an ester of a monohydric alcohol includes diethyl phthalate, dibutyl phthalate, di(2-ethylhexyl) phthalate, dibutyl maleate, dibutyl adipate, di(2-ethylhexyl) sebacate, ethyl myristate, isopropyl myristate, octyldodecyl myristate, isopropyl palmitate, butyl stearate, isopropyl isostearate, hexyl laurate, cetyl lactate, myristyl lactate, octyldodecyl oleate, hexyldecyl dimethyloctanoate, cetyl 2-ethylhexanoate, isocetyl 2-ethylhexanoate, stearyl 2-ethylhexanoate and dioctyl succinate. An ester of a divalent or more alcohol includes propylene glycol dicaprylate, propylene glycol dicaprate, propylene glycol diisostearate, glyceryl monocaprylate, glyceryl tricaprylate, glyceryl tri-2-ethylhexanoate, glyceryl tricaprate, glyceryl trilaurate, glyceryl triisostarate, glyceryl trioleate and trimethylolpropane tri-2-ethylhexanoate. Above all, from the standpoints of good affinity and compatibility with the acrylic copolymer, carboxylic acid ester is used, glycerin fatty acid ester is preferably used and glycerin ester of saturated fatty acid is more preferably used. Those components can be used alone or by combining two or more kinds.

The content of the component that is liquid or paste-like at room temperature in the adhesive layer is that based on 100 parts by mass of the acrylic copolymer, the upper limit is preferably 60 parts by mass, more preferably 50 parts by mass and still more preferably 45 parts by mass and the lower limit is preferably 5 parts by mass, more preferably 10 parts by mass and still more preferably 15 parts by mass. When the content of the component is in the above range, sufficient flexibility can be imparted to the adhesive layer. As a result, the adhesive layer has good skin stickiness, and additionally, the effects that physical skin irritation when peeling from skin is suppressed and good peelability can be realized can be more satisfactorily obtained.

The adhesive layer in the present invention further contains a tackifier and the tackifier contains a rosin ester. Since the adhesive layer contains a rosin ester, excellent stickiness to a skin surface, particularly skin stickiness when bathing, is imparted to the adhesive layer. Examples of the rosin ester include an ester compound of rosin obtained by esterifying unmodified rosin (for example, gum rosin, wood rosin or tall oil rosin) with alcohols, and an ester compound of modified rosin obtained by esterifying modified rosin (for example, a product obtained by modifying unmodified rosin by polymerization, disproportionation, hydrogenation or the like (polymerized rosin, stabilized rosin, disproportionated rosin, completely hydrogenated rosin, partially hydrogenated rosin, chemically modified rosin and the like)) with alcohols.

As the alcohols used in preparing the rosin ester, polyhydric alcohols, for example, dihydric alcohols such as ethylene glycol, diethylene glycol, propylene glycol, neopentyl glycol, trimethylene glycol, tetramethylene glycol, 1,3-butanediol and 1,6-hexanediol; trihydric alcohols such as glycerin, trimethylolethane, trimethylolpropane and triethylolethane; tetrahydric alcohols such as pentaerythritol and diglycerin; and hexahydric alcohols such as dipentaerythritol are suitably used, but the alcohols may be monohydric alcohols such as methanol and ethanol. Furthermore, as the alcohols, amino alcohols such as triethanolamine, tripropanolamine, triisopropanolamine, N-isobutyldiethanolamine and N-n-butyldiethanolamine can be used.

Examples of the rosin ester includes HARITACK PCJ (manufactured by Harima Chemicals), HARITACK SE10 (manufactured by Harima Chemicals), PENSEL D-125 (manufactured by Arakawa Chemical Industries, Ltd.), PENSEL D-135 (manufactured by Arakawa Chemical Industries, Ltd.), SUPERESTER A-100 (manufactured by Arakawa Chemical Industries, Ltd.), SUPERESTER A-115 (manufactured by Arakawa Chemical Industries, Ltd.) and SUPERESTER A-125 (manufactured by Arakawa Chemical Industries, Ltd.).

The softening point of the rosin ester in the present invention is that the upper limit is preferably 160°C, more preferably 140°C and still more preferably 130°C and the lower limit is preferably 50°C, preferably 70°C, still more preferably 100°C and still further preferably 110°C. When the softening point of the rosin ester is in the above range, high adhesive force to a skin surface can be realized. The softening point of the rosin ester is measured according to JIS K 5601-2-2 (ring and ball method).

The content of the rosin ester in the adhesive layer is that, based on 100 parts by mass of the acrylic copolymer, the upper limit is 50 parts by mass, preferably 40 parts by mass and more preferably 30 parts by mass and the lower limit is 3 parts by mass, preferably 5 parts by mass and more preferably 8 parts by mass. When the content of the component is in the above range, the effects that high adhesive force to a skin surface and suppression of the decrease of adhesive force when moisture intrusion, such as when bathing, are more satisfactorily obtained.

As necessary, various additives such as conventional plasticizer, softener, filler, stabilizer, antioxidant, antibacterial agent or fungicide may be appropriately added to the adhesive layer in the tape or sheet for sticking to skin of the present invention in a range that the object of the present invention does not deviate.

The adhesive layer of the present invention is preferably subjected to a crosslinking treatment in order to impart appropriate cohesive force thereto. The crosslinking treatment includes a physical treatment such as γ-ray irradiation or electron beam irradiation, and a chemical crosslinking treatment by an organic peroxide, an isocyanate compound, an organic metal salt, a metal alcoholate, a metal chelate compound, an epoxy compound, a primary amino group-containing compound or the like. Specific examples of the chemical crosslinking treatment include an organic peroxide such as benzoyl peroxide, an isocyanate compound such as tolylene diisocyanate or hexamethylene diisocyanate, an epoxy compound such as glycerin triglycidyl ether or triglycidyl isocyanurate, and a metal chelate compound such as aluminum tris(acetyl acetonate) or ethyl acetoacetate aluminum diisopropylate. Of those, from crosslinking reactivity and easy handleability, an isocyanate compound, a metal alcoholate and a metal chelate compound are preferably used. By conducting the crosslinking treatment, the balance of characteristics such as flexibility and cohesive force of the adhesive layer can be suitably adjusted, and as a result, adhesive residue when peeling can be well suppressed.

The thickness of the adhesive layer is not particularly limited, and can be appropriately set according to uses of the tape or sheet for sticking to skin of the present invention, the applicable parts of skin to which the tape or sheet is stuck. For example, the thickness of the adhesive layer is that the upper limit is preferably 100 μm and more preferably 80 μm and the lower limit is preferably 30 μm and more preferably 50 μm. When the thickness of the adhesive layer is in the above range, the tape or sheet has good skin adhesiveness when sticking to the skin, and additionally the effects that excellent water vapor permeability is obtained and stuffiness, itch sensation and the like can be suppressed even when sticking for a long period of time are more satisfactorily obtained.

The base material used in the present invention is not particularly limited so long as it does not impair flexibility when sticking and has mechanical strength, and various base materials having various structures made of various materials can be used. The structure of the base material includes fiber assemblies such as woven fabric, nonwoven fabric, knitted fabric or paper, films such as a porous film or an air-permeable film, and a sheet-like product such as a foamed body. Of those, materials having appropriate flexibility that is easy to fit with a curved surface of a sticking part are particularly preferred. The base material may be a single layer of a sheet-like material and may be a laminate of sheet-like materials. The material includes a polyurethane polymer such as polyether urethane or polyester urethane, an amide polymer such as polyether polyamide block polymer, an acrylic polymer such as polyacrylate, a polyolefin polymer such as polyethylene, polypropylene or ethylene/vinyl acetate copolymer, and a polyester copolymer such as polyethylene terephthalate.

The thickness of the base material is preferably determined appropriately depending on the material, uses and the like. In general, in the case of using a film as the base material, the upper limit of the thickness is preferably 100 $\mu$m and more preferably 70 $\mu$m, and the lower limit of the thickness is preferably 3 $\mu$m and more preferably 5 $\mu$m. In the case of using fabrics such as knitted fabric or nonwoven fabric, or a paper as the base material, the absolute thickness is sometimes difficult to measure. Therefore, the upper limit of the basis weight is preferably 200 g/m$^2$ and more preferably 150 g/m$^2$, and the lower limit of the basis weight is preferably 5 g/m$^2$ and more preferably 10 g/m$^2$. In the case of using a foamed body as the base material, the upper limit of the thickness is preferably 3000 $\mu$m, more preferably 2000 $\mu$m and still more preferably 1000 $\mu$m, and the lower limit of the thickness is preferably 100 $\mu$m, more preferably 200 $\mu$m and still more preferably 300 $\mu$m.

From the prevention of stuffiness and itch during sticking the tape or sheet for sticking to skin to a skin surface, holes may be formed in the base material. Holes to be formed in the base material can be easily obtained by applying the conventional hole formation technology such as a metal roll, a perforator or laser irradiation. As the method of forming holes in the base material in the tape or sheet for sticking to skin of the present invention, holes may be formed in the base material itself and then the adhesive layer may be laminated thereon, and the adhesive layer may be laminated on the base material and then holes may be formed. In the case of laminating the adhesive layer on the base material and then forming holes, holes penetrating through the base material and the adhesive layer are formed.

The adhesive layer laminated on at least one surface of the base material may be formed on the entire surface of the base material or may be partially formed thereon. In the case of partially forming the adhesive layer on the base material, the adhesive layer is preferably formed in, for example, a dot shape or a stripe shape. In the case of a stripe shape, the stripe shape includes a straight line shape and a wave shape. However, any shape is acceptable so long as a space functioning as a ventiduct of a gas such as water vapor, that is, stripe space, is secured. Above all, a wave shape is preferred from that change with time of a cross-section of the stripe space during sticking to skin is less.

The tape or sheet for sticking to skin of the present invention is suitably used also in uses such as fixation, joining, decoration, protection, support and the like of various mobile devices (wearable devices) and members constituting the mobile devices in the state of sticking to the mobile devices and the members constituting those. More specifically, fixation, joining, decoration, protection, support and the like of the mobile devices and the members constituting those on the skin can be conducted using the tape or sheet for sticking to skin.

In the tape or sheet for sticking to skin of the present invention, to prevent contamination of the surface of the adhesive layer, the exposed surface of the adhesive layer is generally covered with a release liner until use. The release liner can use a release liner used in general adhesive tapes sticking to skin or skin. Specifically, high quality paper, glassine paper or parchment paper or the like, the surface of which being coated with a release agent having release performance such as a silicone resin or a fluorine resin, high quality paper having a resin anchor-coated with a resin, and a polyethylene laminate, the surface of which being coated with a release agent having release performance such as a silicone resin or a fluorine resin can be used.

One aspect of the tape or sheet for sticking to skin, having a release liner is shown in FIG 1. A tape or sheet for sticking to skin 10 of this aspect includes a base material 1 and an adhesive layer 2 formed on the base material 1, and the surface of the adhesive layer 2 is peelably covered with a release liner 3.

Examples

The present invention is further specifically described below by reference to examples, but those examples do not limit the present invention. The terms "parts" and "%" used hereinafter mean "parts by mass" and "mass %", respectively.

(Example 1)

30 Parts of glyceryl tricaprylate as a liquid or paste-like component, 10 parts of a rosin ester (trade name: HARITACK PCJ, manufactured by Harima Chemicals Corporation) as a tackifier and 0.05 parts of a trifunctional isocyanate compound (trade name: CORONATE HL, manufactured by Tosoh Corporation) as a crosslinking agent were mixed with 100 parts of an acrylic copolymer (copolymer composed of isononyl acrylate : 2-methoxyethyl acrylate : acrylic acid = 65 parts : 30 parts : 5 parts (hereinafter sometimes referred to as "copolymer A")) in toluene, and a uniform adhesive solution was prepared.

The solution was applied to the release-treated surface of a release paper (trade name: KP-64 Shiromaru D Marumizu, manufacture by LINTEC Corporation) such that the thickness after drying became 70 μm and dried at 130°C for 3 minutes. Thus, an adhesive layer was formed. The adhesive layer formed was transferred and stuck to one surface of a polyethylene terephthalate film (thickness 38 μm) as a base material, followed by aging at 60°C for 72 hours. Thus, a tape for sticking to skin of the present invention was obtained.

(Examples 2 to 11 and Comparative Examples 1 to 4)

Tapes for sticking to skin of Examples 2 to 11 and Comparative Examples 1 to 4 were obtained in the same manners as in Example 1, except for changing the acrylic copolymer, the liquid or paste-like component, tackifier and crosslinking agent to those shown in Table 1.

[Table 1]

TABLE 1

| | Acrylic copolymer (parts) | | Liquid or paste-like component (parts) | | Tackifier (parts) | | | Crosslinking agent (parts) | Base material |
|---|---|---|---|---|---|---|---|---|---|
| | Component A | Component B | GTC | IPM | PCJ | SE-10 | YS-O105 | C/HL | |
| Example 1 | 100 | - | 30 | - | 10 | - | - | 0.05 | PET 38 μm |
| Example 2 | 100 | - | 5 | - | 10 | - | - | 0.05 | |
| Example 3 | 100 | - | 15 | - | 5 | - | - | 0.05 | |
| Example 4 | 100 | - | 15 | - | 10 | - | - | 0.05 | |
| Example 5 | 100 | - | 15 | - | 30 | - | - | 0.05 | |
| Example 6 | 100 | - | 15 | - | 40 | - | - | 0.05 | |
| Example 7 | 100 | - | 15 | - | 50 | - | - | 0.05 | |
| Example 8 | 100 | - | 30 | - | 10 | - | - | 0.05 | Urethane 30 μm |

(continued)

| | Acrylic copolymer (parts) | | Liquid or paste-like component (parts) | | Tackifier (parts) | | | Crosslinking agent (parts) | Base material |
|---|---|---|---|---|---|---|---|---|---|
| | Component A | Component B | GTC | IPM | PCJ | SE-10 | YS-O105 | C/HL | |
| Example 9 | 100 | - | 40 | - | 10 | - | - | 0.05 | PET 38 μm |
| Example 10 | 100 | - | 30 | - | - | 10 | - | 0.05 | |
| Example 11 | 100 | - | - | 30 | 10 | - | - | 0.05 | |
| Comparative Example 1 | 100 | - | - | - | 10 | - | - | 0.05 | |
| Comparative Example 2 | 100 | - | 30 | - | - | - | - | 0.05 | |
| Comparative Example 3 | - | 100 | 30 | - | 10 | - | - | 0.05 | |
| Comparative Example 4 | 100 | - | 30 | - | - | - | 10 | 0.05 | |

Component A: Acrylic copolymer (copolymer composed of isononyl acrylate : 2-methoxyethyl acrylate : acrylic acid = 65 parts : 30 parts : 5 parts)
Component B: Acrylic copolymer (2-ethylhexyl acrylate : acrylic acid = 95 parts : 5 parts)
GTC: Glyceryl tricaprylate
IPM: Isopropyl myristate
PCJ: Rosin ester (trade name: HARITACK PCJ, manufactured by Harima Chemicals Corporation)
SE-10: Rosin ester (trade name: HARITACK SE-10, manufactured by Harima Chemicals Corporation)
YS-O105: Aromatic modified terpene resin (trade name: YS-O105, manufactured by Yasuhara Chemicals)
C/HL: Coronate HL (manufactured by Tosoh Corporation)
PET: Polyethylene terephthalate film
Urethane: Polyurethane film

The following tests were conducted using the tape for sticking to skin obtained in each example and comparative example.

(Adhesive force to collagen film in dry state)

The tape for sticking to skin was cut into a ribbon shape having a width of 10 mm and a length of 50 mm, and the release paper was peeled to expose an adhesive surface. The adhesive surface was press-adhered to a collagen film (width 25 mm, length 100 mm, thickness 38 μm) by reciprocating one time a 2 kg roller. After the elapse of 30 minutes at room temperature in this state, the tape for sticking to skin was peeled in 180° direction in a peel rate of 300 mm/min, and the peel force in the peeling was measured. The same test was repeated three times, and the average value of peel forces of three tests was taken and was calculated as the value of an adhesive force ($P_{dry}$) to a collagen film in dry state.

(Adhesive force to collagen film in wet state)

The tape for sticking to skin was cut into a ribbon shape having a width of 10 mm and a length of 50 mm, and the release paper was peeled to expose an adhesive surface. The adhesive surface was press-adhered to a collagen film (width 25 mm, length 100 mm, thickness 38 μm) by reciprocating one time a 2 kg roller. Next, purified water was poured on the surface of the collagen film on which the tape for sticking to skin was not stuck in an amount sufficiently covering the entire surface. After the elapse of 30 minutes in this state, the poured purified water was wiped with a waste, the tape for sticking to skin was peeled in 180° direction in a peel rate of 300 mm/min, and the peel force in the peeling was measured. The same test was repeated three times, and the average value of peel forces of three tests was taken and was calculated as the value of an adhesive force ($P_{wet}$) to collagen film in wet state.

(Rate of change of adhesive force)

The change rate of adhesive force was calculated from the above values of the adhesive force to the collagen film in the dry state and wet state by the following formula.

$$\text{Rate of change of adhesive force} = (P_{dry} - P_{wet})/P_{dry} \times 100 \ (\%)$$

(Adhesive force to sebum)

Model sebum (glyceryl tricaplyrate) was diluted with toluene so as to be a concentration of 40 mg/mL. 100 $\mu$L of the diluted liquid was applied to the surface of a collagen film (width 25 mm, length 100 mm, thickness 38 $\mu$m) so as to be uniform thickness and air-dried to remove toluene, and the collagen film having the model sebum applied thereon was prepared. Next, a tape for sticking to skin was cut into a ribbon shape having a width of 10 mm and a length of 50 mm, and the release paper was peeled to expose an adhesive surface. The adhesive surface was press-adhered to a collagen film having the model sebum applied thereto by reciprocating one time a 2 kg roller. After the elapse of 30 minutes at room temperature in this state, the tape for sticking to skin was peeled in 180° direction in a peel rate of 300 mm/min, and the peel force in the peeling was measured. The same test was repeated three times, and the average value of peel forces of three tests was taken and was calculated as the value of adhesive force to sebum.

(Underwater constant load peelability)

Underwater constant load peelability is described by reference to FIG 2.
Collagen film 22 (width 25 mm, length 100 mm, thickness 38 $\mu$m) was stuck to the surface of a bakelite plate 23 (width 30 mm, length 130 mm, thickness 2 mm) through a double-sided tape (not shown), and a test plate was prepared. Next, a tape for sticking to skin 21 was cut into a ribbon shape having a width of 10 mm and a length of 50 mm, and the release paper was peeled to expose an adhesive surface. The adhesive surface was press-adhered to the collagen film 22 of the test plate by reciprocating one time a 2 kg roller, and a test sample was obtained. After the elapse of 30 minutes at room temperature in this state, a support 27 having a shape shown in FIG 2 was installed in a tank 25 with 40°C water 24. The test sample was arranged so as to be placed on the support 27 in the state that separated two edges of the bakelite plate 23 are shown in FIG 2, 60 g of a weight 26 was hung on one edge of the tape for sticking to skin 21, and distance and time that the tape for sticking to skin 21 was peeled were measured. The same test was repeated three times, and peel rates were calculated by the following formula. The average value was taken and calculated as the value of underwater constant load peelability. Smaller value indicates that the tape is difficult to peel in water.

$$\text{Peel rate (mm/min)} = \text{Peeled distance (mm)/peel time (min)}$$

(Adhesive force to human skin)

Each of the tapes for sticking to skin of Example 1 and Comparative Examples 1 to 4 was cut into a ribbon shape having a width of 10 mm and a length of 50 mm, and the release paper was peeled to expose an adhesive surface. The adhesive surface was press-adhered to a forearm of a volunteer by reciprocating one time a 2 kg roller. After the elapse of 30 minutes at room temperature in this state, the tape for sticking to skin was peeled in 180° direction in a peel rate of 300 mm/min, and the peel force in the peeling was measured. The same test was repeated three times, and the average value of peel forces of three tests was taken and was calculated as the value of an adhesive force to skin.

(Anchorability)

During or after measuring adhesive force to collagen film (dry state, wet state), adhesive force to sebum and underwater constant load peelability, the state of anchorability was visually observed, and the anchorability was evaluated by the following criteria.

○: No anchor destruction
△: Anchor destruction is slightly observed
×: Anchor destruction is apparently observed

The results obtained are shown in Table 2.

[Table 2]

TABLE 2

| | | | | | Results | | |
|---|---|---|---|---|---|---|---|
| | $P_{dry}$ (N/10 mm) | $P_{wet}$ (N/10 mm) | Rate of Change of adhesive force (%) | Adhesive force to sebum (N/10 mm) | Underwater constant load peelability (N/10 mm) | Anchorability | Adhesive force to human skin (N/10 mm) |
| Example 1 | 2.2 | 0.7 | 68 | 3.2 | 15.9 | ○ | 5.1 |
| Example 2 | 3.1 | 0.2 | 94 | 4.1 | 3.1 | ○ | - |
| Example 3 | 2.4 | 0.4 | 83 | 3.6 | 6.9 | ○ | - |
| Example 4 | 2.4 | 0.4 | 83 | 3.5 | 8.2 | ○ | - |
| Example 5 | 2.8 | 0.4 | 86 | 4.1 | 4.1 | ○ | - |
| Example 6 | 3.5 | 0.4 | 89 | 5.2 | 2.9 | ○ | - |
| Example 7 | 3.7 | 0.3 | 92 | 6.2 | 2.2 | ○ | - |
| Example 8 | 2.1 | 0.4 | 81 | 3.5 | 11.7 | ○ | - |
| Example 9 | 2.4 | 1.1 | 54 | 5.4 | 20.4 | ○ | - |
| Example 10 | 2.0 | 0.9 | 58 | 3.8 | 16.8 | ○ | - |
| Example 11 | 2.2 | 0.9 | 59 | 4.3 | 11.4 | Δ | - |
| Comparative Example 1 | 3.4 | 0.1 | 97 | 4.5 | 1.6 | ○ | 1.4 |
| Comparative Example 2 | 1.8 | 0.7 | 61 | 2.7 | 22.4 | ○ | 3.0 |
| Comparative Example 3 | 1.1 | 0.6 | 45 | 1.5 | 3.2 | ○ | 1.4 |
| Comparative Example 4 | 2.3 | 0.8 | 65 | 4.0 | 14.6 | × | 3.1 |

As shown in Table 2, it was confirmed that the tape for sticking to skin of Example 1 has high adhesive force to human skin and excellent skin stickiness as compared with the tapes for sticking to skin of Comparative Examples. Furthermore, it was confirmed that the tape for sticking to skin of each Example shows good results in each evaluation item, and particularly has good adhesive characteristics in wet state in which water is present, as compared with the tape for sticking to skin of each Comparative Example.

On the other hand, the tape for sticking to skin of Comparative Example 1 had low adhesive force to human skin and large decrease in the change of adhesive force, and was poor in adhesive characteristics.

The tape for sticking to skin of Comparative Example 2 had low adhesive force to sebum and large value of underwater constant load peelability. Therefore, the tape was easy to peel and was poor in adhesive characteristics.

The tape for sticking to skin of Comparative Example 3 had low adhesive force to human skin and adhesive force to sebum, and was poor in adhesive characteristics.

The tape for sticking to skin of Comparative Example 4 generated apparent anchor destruction.

Industrial Applicability

According to the present invention, a tape or sheet for sticking to skin that has high adhesive force to skin and excellent skin stickiness, and can suppress great deterioration of adhesive force to an adherend on which water such as sweat generated during bathing or from skin and obtain good adhesiveness can be provided.

Reference Signs List

1: Base material
2: Adhesive layer
3: Release liner
10: Tape or sheet for sticking to skin
21: Tape for sticking to skin
22: Collagen film
23: Bakelite plate
24: Water
25: Tank
26: Weight
27: Support

**Claims**

1. A tape or sheet for sticking to skin, which comprises a base material and an adhesive layer laminated on at least one surface of the base layer,

   wherein the adhesive layer contains an acrylic copolymer obtained by copolymerizing a monomer mixture including a (meth)acrylic acid alkyl ester monomer and an alkoxy group-containing ethylenically unsaturated monomer, a component that is liquid or paste-like at room temperature, and a tackifier,
   wherein the tackifier includes a rosin ester,
   wherein the component that is liquid or paste-like at room temperature includes a carboxylic acid ester, and
   wherein a content of the rosin ester in the adhesive layer is 3 parts by mass or more and 50 parts by mass or less based on 100 parts by mass of the acrylic copolymer.

2. The tape or sheet for sticking to skin according to claim 1, wherein the carboxylic acid ester includes a glycerin ester of a saturated fatty acid.

3. The tape or sheet for sticking to skin according to claim 1 or claim 2, wherein a softening point of the rosin ester is 50 to 160°C, the softening point being measured according to JIS K 5601-2-2 (ring and ball method).

**Patentansprüche**

1. Ein Band oder eine Folie zum Aufkleben auf die Haut, umfassend ein Grundmaterial und einer auf mindestens eine Oberfläche der Grundschicht laminierte Klebeschicht,

   wobei die Klebeschicht ein Acrylcopolymer enthält, das durch Copolymerisation einer Monomermischung erhalten wird, die ein (Meth)acrylsäurealkylestermonomer und einen alkoxygruppenhaltiges ethylenisch ungesättigtes Monomer, eine bei Raumtemperatur flüssige oder pastöse Komponente und einen Klebrigmacher umfasst, wobei der Klebrigmacher einen Kolophoniumester enthält,
   wobei die bei Raumtemperatur flüssige oder pastöse Komponente einen Carbonsäureester enthält, und
   wobei der Gehalt des Kolophoniumesters in der Klebeschicht bezogen auf 100 Massenteile des Acrylcopolymers 3 Massenteile oder mehr und 50 Massenteile oder weniger beträgt.

2. Das Band oder die Folie zum Aufkleben auf die Haut gemäß Anspruch 1, wobei der Carbonsäureester einen Glycerinester einer gesättigten Fettsäure enthält.

3. Das Band oder die Folie zum Aufkleben auf die Haut gemäß Anspruch 1 oder Anspruch 2, wobei der Erweichungspunkt des Kolophoniumesters 50 bis 160 °C beträgt und der Erweichungspunkt gemäß JIS K 5601-2-2 (Ring- und Kugelmethode) gemessen wird.

**Revendications**

1. Ruban ou feuille permettant d'adhérer à la peau, qui comprend un matériau de base et une couche adhésive stratifiée sur au moins une surface de la couche de base,

   dans lesquels la couche adhésive contient un copolymère acrylique obtenu par copolymérisation d'un mélange de monomères comportant un monomère ester alkylique d'acide (méth)acrylique et un monomère à insaturation éthylénique contenant un groupe alcoxy, un constituant qui est liquide ou pâteux à température ambiante, et un agent collant,
   dans lesquels l'agent collant comporte un ester de colophane,
   dans lesquels le constituant qui est liquide ou pâteux à température ambiante comporte un ester d'acide carboxylique, et
   dans lequel une teneur de l'ester de colophane dans la couche adhésive est de 3 parties en masse ou plus et 50 parties en masse ou moins sur la base de 100 parties en masse du copolymère acrylique.

2. Ruban ou feuille permettant d'adhérer à la peau selon la revendication 1, dans lesquels l'ester d'acide carboxylique comporte un ester de glycérine d'un acide gras saturé.

3. Ruban ou feuille permettant d'adhérer à la peau selon la revendication 1 ou la revendication 2, dans lesquels un point de ramollissement de l'ester de colophane va de 50 à 160 °C, le point de ramollissement étant mesuré conformément à la JIS K 5601-2-2 (méthode de l'anneau et de la bille).

*FIG. 1*

*FIG. 2*

(a)

(b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007209515 A
- EP 1184039 A2
- US 2009274748 A1